# EUROPEAN PATENT SPECIFICATION

(11) **EP 1 325 963 B1**
(45) Date of publication and mention of the grant of the patent: **27.09.2006**
(21) Application number: 01811266.4
(22) Date of filing: 24.12.2001
(51) Int. Cl.: C12Q 1/68

(54) **Method for non-invasive diagnosis of transplantations and transfusions**
Verfahren zur nicht-invasiven Diagnose von Transplantationen und Transfusionen
Procede de diagnostic non- invasif des transplantations et transfusions

(43) Date of publication of application: 09.07.2003
(73) Proprietor: Holzgreve, Wolfgang, Prof., 4051 Basel (CH); Hahn, Sinuhe, Dr., 4410 Liestal (CH); Garritsen, Hendrikus Stephanus Paulus, 48149 Münster (DE)
(72) Inventor: Holzgreve, Wolfgang, Prof., 4051 Basel (CH); Hahn, Sinuhe, Dr., 4410 Liestal (CH); Garritsen, Hendrikus Stephanus Paulus, 48149 Münster (DE)
(74) Representative: Eder, Carl E.

(56) References cited:
- GARRITSEN H S P ET AL: "A sequence-specific polymerase chain reaction assay for mitochondrial DNA polymorphisms in human platelets and white cells." TRANSFUSION (BETHESDA), vol. 37, no. 10, 1997, pages 1012-1019, XP001062896 ISSN: 0041-1132
- GARRITSEN HENK S P ET AL: "Identifying allogeneic platelets by resolution of point mutations in mitochondrial DNA using single-stranded conformational polymorphism PCR." TRANSFUSION (BETHESDA), vol. 41, no. 12, December 2001 (2001-12), pages 1531-1538, XP001062898 ISSN: 0041-1132
- GARRITSEN H S P ET AL: "Mitotyping: A new sensitive method for microchimerism detection." EUROPEAN JOURNAL OF IMMUNOGENETICS, vol. 28, no. 2, April 2001 (2001-04), page 209 XP001057182 15th European Histocompatibility Conference;Granada, Spain; March 27-30, 2001 ISSN: 0960-7420
- GARRITSEN H S P ET AL: "Polymorphisms in the non-coding region of the human mitochondrial genome in unrelated plateletapheresis donors." BRITISH JOURNAL OF HAEMATOLOGY, vol. 112, no. 4, March 2001 (2001-03), pages 995-1003, XP001038290 ISSN: 0007-1048
- GARRITSEN H S P ET AL: "A sequence specific PCR (SSP-PCR) assay for DNA polymorphisms in mitochondria." TRANSFUSION (BETHESDA), vol. 36, no. 9 SUPPL., 1996, page 59S XP001062895 49th Annual Meeting of the American Association of Blood Banks;Orlando, Florida, USA; October 12-16, 1996 ISSN: 0041-1132

## Description

Clinical solid organ and allogeneic stem cell transplantation implies the Transplantation of a functional unit of allogeneic origin to a diseased patient. This functional unit can either be accepted or rejected by the transplant recipient. There are multiple parameters such as functional status of the organ, immunological compatibility etc., influencing the final acceptance of the organ or stem cell engraftment. Pharmacological support after transplantation can modulate the immune system of the recipient and thereby reduce transplant rejection.

Due to the acute shortage of suitable donor organs or stem cell donors, it is extremely important to minimise loss due to rejection. The outcome of transplantation is improved by an extensive pre-transplant compatibility testing program and by continuously monitoring of the functional status of the transplant and any signs of rejection. This form of monitoring is also useful in adjusting the dosage of the immunosuppression treatment necessary for graft acceptance.

A relevant issue in this monitoring is not only what should be measured but also where it should be measured.

One strategy that has been created is to measure immune responses specific for the antigens in question, such as foreign class I and class II MHC in the case of allografts, at the site of the graft (tissue biopsy). This invasive diagnostic procedure has the following disadvantages:
- It influences the quality of life of the patient negatively (hospital stay).
- It is associated with complications such as hematuria, anuria, perirenal hematoma, bleeding and shock, arteriovenous fistula and graft loss in the case of renal transplantation. In liver and heart transplantation similar complications have been reported.
- There exist a limited amount of biological material and required reagents and there is only a limited number of biopsies possible.
- The rejection can be a focal problem in an organ: sampling errors cause false negative results. Therefore multiple biopsy samples have to be taken to increase the diagnostic accuracy, which are associated with a high risk of transplant loss.
- A very high overall cost of invasive procedures.

Based on this knowledge another strategy has been created and lead to the development of non-invasive diagnostic procedures to measure rejections. Such procedures include:
a) Monitoring of the types of immune cells present in the circulation at a given time.
b) Measuring shifts in cytokine patterns.
c) Measuring mRNA coding for perforin and granzyme B, cytotoxic proteins associated with organ rejection.

The advantages of these non-invasive diagnostic techniques are that they do not interfere with the quality of life of the transplant recipient, in general only a blood or urine sample is required and tests can be standardised and/or automated. Further hardly no procedural complications are associated with non-invasive testing methods and these methods are cost-effective in comparison to invasive procedures.

The disadvantages of the known non-invasive tests are that they require specialised technologists in order to obtain valid results, that the tests based on mRNA-analysis are influenced by the very short lifetime of this molecule and therefore requires a very rapid processing of samples, that the monitoring of immune cells and or inflammatory cytokine proteins is hampered by possible infections which can cause similar alterations as are present under conditions of transplant rejection, and that none of these traits have proven to be useful in identifying early stages of graft rejection.

Apart from donor specific proteins and genomic DNA, it is also known, that by tissue damage of the donor graft mito-chondrial DNA (mtDNA) will be released into the circulation of the recipient. With the exception of mature erythrocytes, all cellular blood components contain mitochondria. Thousands of mitochondria per cell are present in leukocytes, and a mean of 3-4 mitochondria are also present in platelets. Each of these mitochondria contain at least one copy of mtDNA. Mitochondrial DNA encodes for a complete set or ribosomal RNA and transfer RNA as well as 13 polypeptides of oxidative phosphorylation. This circular 16,6 kb human mtDNA was completely sequenced in 1981.

TRANSFUSION vol. 37, 1997, p. 1012-1019 and EUR. J. IMMUNOGENETICS vol. 28, no. 2, April 2001, p. 209; and 15^{th} European Histocompatibility Conference, Granada, Spain, March 27.30, 2001, describe SSP-PCR in order to detect donor-specific mtDNA in an *in vitro* system mimicking the recipient's blood composition after platelet transfusion, the sequence-specific primers ASF and BSF being located in the hypervariable stretches of mtDNA.
TRANSFUSION vol. 41/12, December 2001, p. 1531-1538; and BRIT. J. HAEMATOL. vol. 112, no. 4, March 2001, p. 995-1003 describe analysis of HVR 1 and HVR 2 regions of mtDNA for polymorphisms by means of SSCP-PCR or sequence analysis, both methods being said to be useful for the diagnosis of transfusion by means of detecting donor-specific mtDNA and having been tested in an in vitro system.

It is the object of the present invention to suggest a method or process to measure the quantity of cell free circulatory mtDNA in bodily fluids as an indicator or marker of tissue damage and to use mtDNA polymorphisms to discriminate between donor and recipient tissue damage in a transplant setting. Furthermore, the method should not include labelling of cellular blood products because this could potentially modify the products and/or affect their quality, and the method should be easy to perform.

This stated object of the invention is achieved by the method according to claim 1.

The method according to the invention is a non-invasive diagnostic procedure characterised by the following steps:
a) Extracting cell free circulatory mitochondrial DNA (mtDNA) from a probe of cell free bodily fluids from a transplantation or transfusion recipient;
b) Detecting, analysing and quantifying mtDNA from the probe;
c) Comparing mtDNA from the probe with mtDNA obtained from donor and recipient before transplantation or transfusion;
d) Using mtDNA polymorphisms in hypervariable regions to discriminate between donor tissue damage and recipient tissue damage in a transplant setting or to determine survival of cellular blood components in a transfusion.

Advantages in this procedure compared to other non-invasive methods are:
- It specifically detects damage of the target organ as there is no other source of mtDNA containing the specific polymorphisms.
- It uses double stranded DNA which is much more stable in the circulation than mRNA.
   It uses mtDNA which is present at over 10'000 copies on a per cell basis. This increases the sensitivity 2-3 logs when compared to a single copy nuclear genomic single nucleotide polymorphism marker.
- It uses the hypervariable region of mitochondrial DNA. This D-loop region is present in a number of copies per single mtDNA molecule. This provides an additional number of copies of the target sequence per cell.
- Mutation rate of mitochondrial DNA is 10-20 x higher than genomic DNA thereby increasing the likelyhood of finding informative polymorphisms.
- The mtDNA sequence has been described and many polymorhisms have been characterised.

It is known that human mtDNA represents an unique target for detecting allogenic transfused platelets. By sequencing 100 platelet apherese donors there have been found over 20 frequent single nucleotide polymorphisms (SNP's) in the control region of mtDNA. The present invention discloses the possibility of targeting these polymorphisms with PCR using sequence specific primers (SSP) and in this way, an adapted new technique to discriminate allogeneic cells from autologous cells. Specifically, new sequence specific primer pairs have been designed and PCR-conditions optimised.

Therefore an other object of the invention is providing the method with a SSR-PCR system for frequent DNA polymorphisms, sequence specific primer pairs and an amplification control primer pair to indicate whether the PCR-reaction took place.

It is a further object of the invention to provide a SSP-PCR kit which detects frequent polymorphisms in the human mtDNA. This kit can be used to identify informative markers for chimerism and survival studies after transfusions of cellular blood components.

Advantageous embodiments of the invention become evident from the dependent claims.

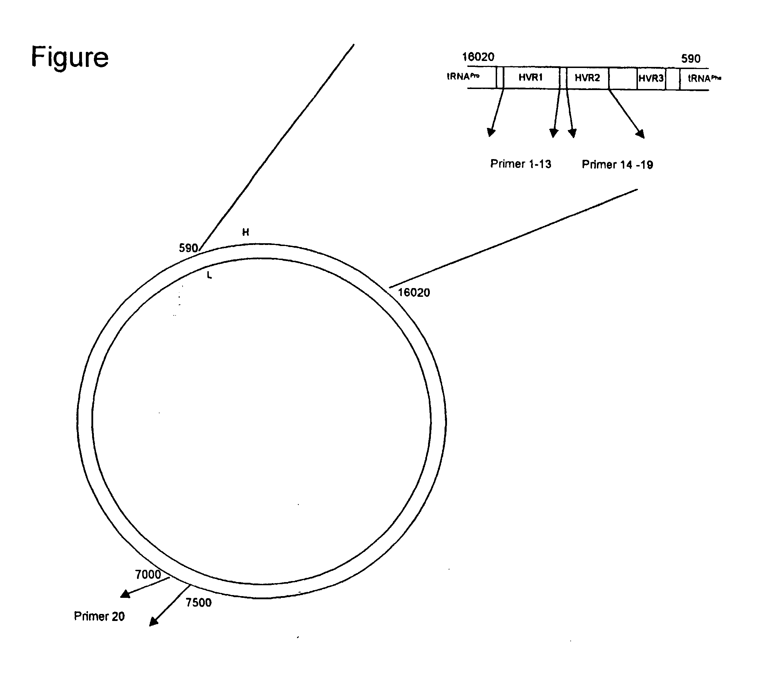

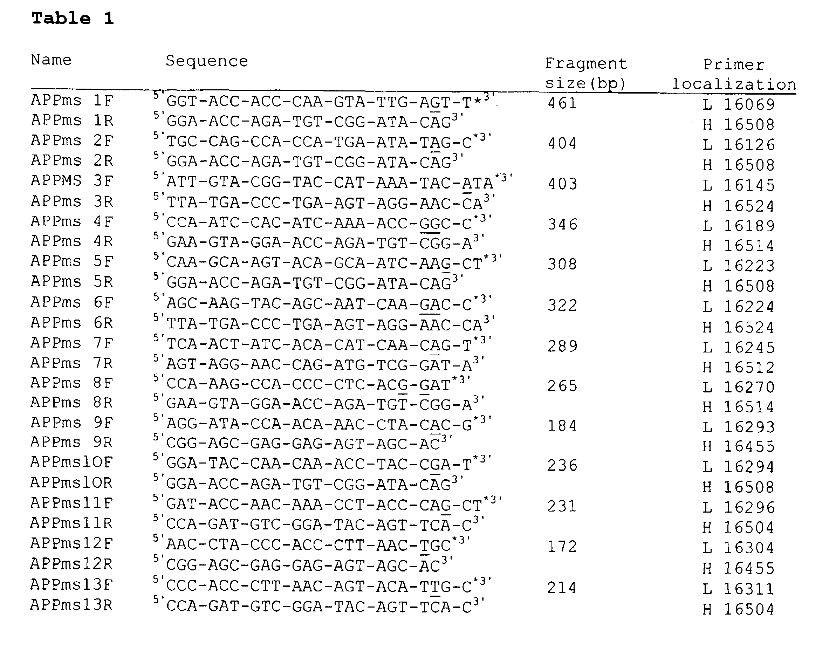

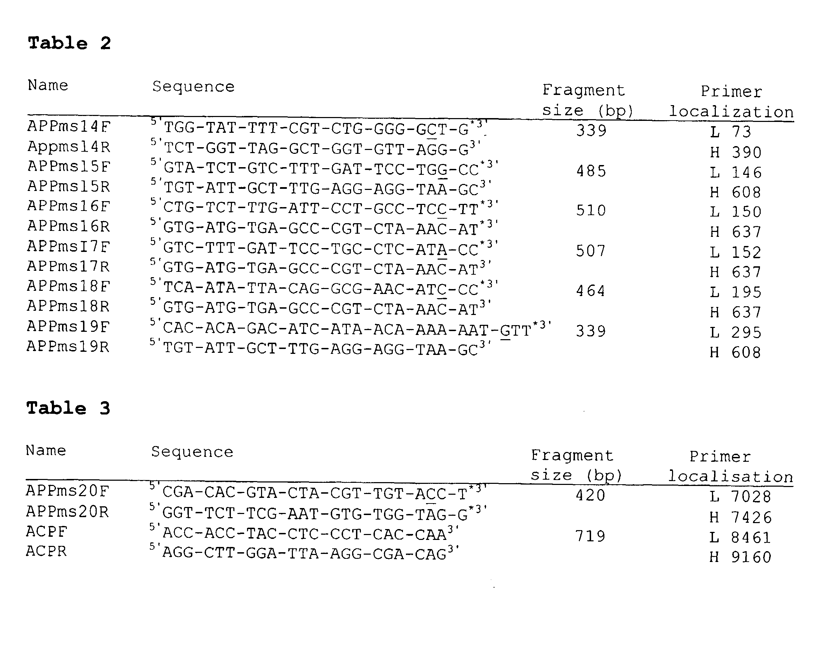

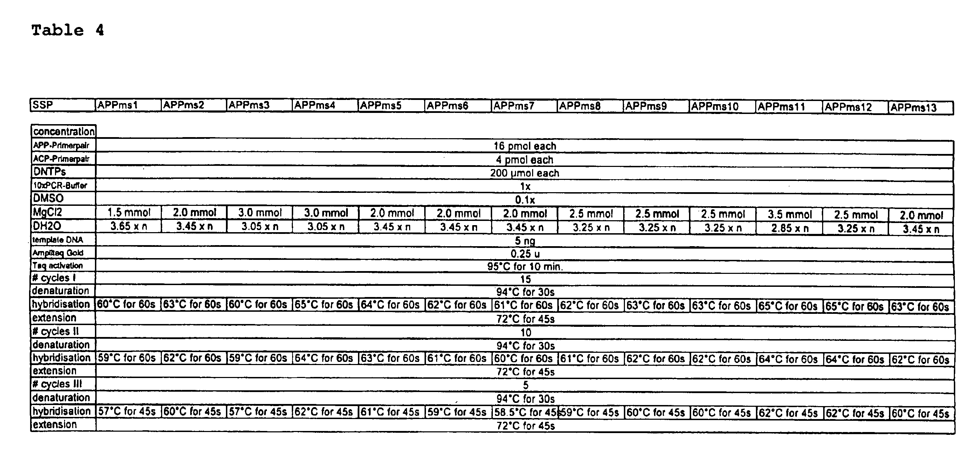

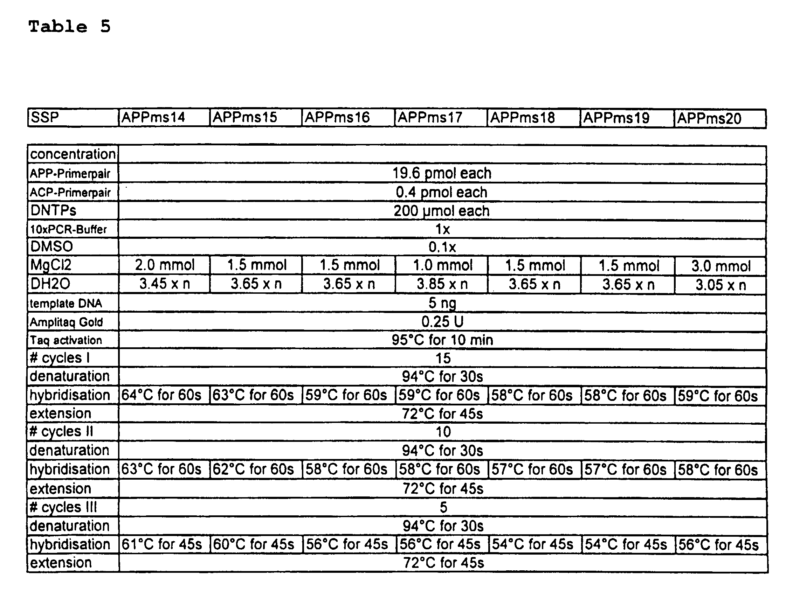

The present invention will now be illustrated in more detail by the following description with reference to the enclosed drawing. In the drawing,

The Figure shows a schematic overview of the localisation of the SNP's within the human mtDNA,
Table 1 shows the oligonucleotide primers for HVRI Mitotyping by PCR-SSP,
Table 2 shows the oligonucleotide primers for HVR2 Mitotyping by PCR-SSP,
Table 3 discloses the oligonucleotide primers for COXI mitotyping and control primer by PCR-SSP,
Table 4 shows the thermal profile and PCR-conditions for HVRI primer pairs, and
Table 5 shows the thermal profile and PCR-conditions for HVR2 primer pairs.

Using two well known mtDNA polymorphisms, it is possible to establish a sequence specific primer-PCR (SSP-PCR) system for the detection of transfused allogeneic thrombocytes. However, these two primers detect infrequent mtDNA polymorphisms in the caucasian population and are not very informative in practice. To increase the number of informative primers, it has to be turned to two highly polymorphic regions (HVRI and HVR2) within the 1,024 bp D-loop region. This D-loop region is a non-coding region which contains regulatory elements for replication and transcription.

The figure of the drawing gives a schematic overview of the localisation of the SNP's within the human mtDNA targeted by sequence specific primers. The circular mitochondrial genome is shown in the lower left of the figure. L represents the light strand and H the heavy strand. Reading counter clockwise at the lower left of the figure the region coding for cytochrome c oxidase I (COX I) is shown (between bp 7000-7500), containing primer pair 20. At the upper right of the figure the D-loop region is presented schematically. The D-loop is bound by the genes for tRNA proline (tRNAPro) and tRNA phenylalanine (tRNAPhe). The positions of the different hypervariable regions are shown. 13 primer pairs are located within the first hypervariable region (HVR1) of the D-loop region. Six primer pairs are situated in the second hypervariable region (HVR2) and one primer pair is located within the sequence coding for cytochrome c oxidase I (COX I).

The SNP's are selected based on a nucleotide sequence analysis of 100 platelet apheresis donors. All selected SNP's are expressed in over 10 percent of the donors. In addition, the polymorphism at np 7025 is chosen because it is expressed by 34% of caucasians. Both forward and reverse primers can be designed with the assistance of primer design software available on the Internet under http//www.williamstone.com. Oligonucleotide primers can be analysed for potentially interfering secondary structures, such as hairpin loops and differences in melting temperature. The nucleotide sequences and localisation of the primers used for PCR-SSP mitotyping are presented in tables 1 to 3.

In the tables localisation, fragment size and sequence of primers used for sequence specific amplification of mitochondrial template DNA are shown. F stands for forward primer and R stand for reverse primer. The primers are numbered according to the location of their 5' end in the reference sequence (Anderson et al. Nature, 1981, 290, 457 - 465). H stands for the heavy strand, which is purine rich. L stands for the (pyrimidine rich) light strand. *indicates the allele differentiating base. The bases mismatched with their target template are underlined.

In each PCR-reaction a primer pair is included which amplifies a conserved sequence in the mtDNA and thus functions as an amplification control primer. The concentration of the control primer pair is kept 4 to 20-fold lower than the concentration of the allele-specific primers in order to increase specificity and avoid false negative typing results due to competitive priming.

The specific amplification conditions and reaction mixtures for each PCR-SSP reaction are described in Tables 4 and 5. The total PCR-reaction volume is 10 µl. Each PCR - reaction mixture contains one SSP-primer pair (tables 1-3) and the internal positive control primer pair (table 3). For specific concentration of the primer pairs see tables 4 and 5. In addition to the primers, the PCR-reaction mixtures also contain template mtDNA, PCR, buffer, dNTP's, Dimethylsulfoxide, Taq Polymerase and Magnesiumchloride. Because each PCR-reaction has to be optimised for each of the components, the concentration of the different components and annealing temperatures vary. The exact concentrations of the different components can be found in tables 4 and 5. The 10 µl reaction volume is completed by adding bidistilled water.

PCR can be performed using a programmable thermal cycler. Further 10 µl of amplified product can be visualised and analysed for the presence or absence of PCR products using a 2 percent agarose gel with ultraviolet illumination after ethidium bromide staining.

Results of experiments according with the above described conditions are described below:

In fourty cases, positive PCR-SSP reactions in the template mtDNA of previously untested individuals were found and confirmed by automated sequencing of the template mtDNA in an independent sequencing facility.

### Detection of allogeneic leukocytes

Each nucleated cell contains thousands of mitochondria. Theoretically, this would indicate that more template mtDNA than genomic DNA is available on a per cell basis. In order to investigate whether this could be proven, two SSP-PCR systems have been compared. One is a SSP-PCR system for genomic DNA (HLA-B low Kit, Genovision, Vienna, Austria), and the other is the mitotyping SSP-PCR system for mtDNA according to the invention. Mononuclear cells from 6 individuals were isolated by density gradient centrifugation, from which genomic polymorphisms (HLA-B type) and mitochondrial polymorphisms (mitotyping) were known. To simulate donor/recipient combinations, six pairs were prepared with a 10-fold serial dilution of donor cells. Starting point was 10⁶ mononuclear cells. DNA was extracted using a commercial kit (Puregene, gentra systems) and PCR was performed. The amount of DNA and cycling conditions (30 cycles) were kept similar for both systems. 10 µl of amplified product was visualised and analysed for the presence or absence of PCR-products using a 2 percent agarose gel with ultraviolet illumination after ethidium bromide staining.

It has been found that in all six combinations, donor specific sequences detected by mtDNA specific primers were 102 to 103-fold more sensitive compared to primers detecting HLA-polymorphisms.

### Detection of transfused allogeneic platelets

Platelets contain only a very small number of mitochondria. This is fully compensated by the large amounts of platelets (10¹²/unit) transfused. To investigate the detection limit of the developed SSP-PCR system for mitochondrial DNA in this application, a dilution series of isolated single donor platelets from 6 individuals with known mtDNA polymorphisms has been prepared. To simulate donor/recipient combinations, six pairs were prepared with a 10-fold serial dilution of donor cells. Starting point was 10⁸ platelets. DNA was extracted using a commercial kit and PCR was performed with a primer pair indicative of the informative polymorphism. Cycling conditions are identical to the conditions mentioned before. 10 µl of amplified product was visualised and analysed for the presence or absence of PCR-products using a 2 percent agarose gel with ultraviolet illumination after ethidium bromide staining.

Using 10⁸ platelets a reactivity endpoint of 10² - 10³ for different informative primers has been analysed, which means that with the method according to the invention 100 - 1000 allogeneic platelets in a background of 100 million platelets can be detected.

In two cases the above mentioned second experiment has been extended by mixing precounted amounts of allogeneic platelets (10-fold dilution series, starting point 10⁶ platelets) into a tube of EDTA blood of a recipient. This in order to imitate a platelet transfusion. After mixing for 15 minutes on a roler, platelet rich plasma was isolated by density centrifugation. DNA was extracted using a commercial kit and PCR was performed with a primer pair indicative of the informative polymorphism. Cycling conditions are identical to the conditions mentioned above. 10 µl of amplified product were visualised and analysed for the presence or absence of PCR-products using a 2 percent agarose gel with ultraviolet illumination after ethidium bromide staining. Similar to the platelet mixtures, a sensitivity of 10² or 10³ allogeneic platelets could be achieved in these whole blood mixtures.

Specificity was also tested by taking 10 randomly selected individuals and screening them for the 20 frequent mtDNA polymorphisms. The next step was to make pairs of these individuals and to see if one could discriminate between them based upon the screening results. In 94% of the combinations they could be identified using the inventive mitotyping set of primers.

As demonstrated above, the use of SNPS's in mitochondrial DNA as a potential target according to the invention is feasible and covers most of the issues already mentioned. In addition to the target, the selection of the molecular genetic amplification technique is also of extreme importance. According to the invention the Sequence Specific Primer-PCR technique has been chosen. PCR-SSP systems featuring multiple, small volume PCR-reactions where each reaction is specific for a single nucleotide polymorphism. PCR-SSP specificity is derived from matching the terminal 3' nucleotide of the primers with the target DNA-sequence. Taq polymerase can extend 3'-matched primers but not 3'-mismatched primers, consequently only target DNA complementary to both primers is efficiently amplified. PCR-SSP works because Taq polymerase lacks 3' to 5'- exonucleolytic proof-reading activity. Thus the 3' mismatch principle can be used to identify virtually any single point mutation within one or two PCR-SSP reactions. This technique has also been chosen because it has major advantages in comparison to other molecular genetic techniques: It is easy to perform with minimal technical expenditure, and the post-amplification detection is minimal. Further, the system can be easily adapted to new polymorphisms.

The method according to the invention also has its limitations: Because of the maternal inheritance of mtDNA, cells derived from maternally related individuals cannot be discriminated from each other. Furthermore mature erythrocytes carry no mitochondria and have to be excluded from the analysis.

## Claims

1. A method for the non-invasive diagnosis of transplantations and transfusions **characterised by**
a) Extracting cell free circulatory mitochondrial DNA (mtDNA) from a probe of cell free bodily fluids from a transplantation or transfusion recipient;
b) Detecting, analysing and quantifying mtDNA from the probe;
c) Comparing mtDNA from the probe with mtDNA obtained from donor and recipient before transplantation or transfusion;
d) Using mtDNA polymorphisms in hypervariable regions to discriminate between donor tissue damage and recipient tissue damage in a transplant setting or to determine survival of cellular blood components in a transfusion.

2. The method according to claim 1, **characterised in that** detecting, analysing and quantifying mtDNA in the probe of the recipient is done by Taqman probes, molecular beacons or other means of real time PCR, gene array, or SSCP densitometry.

3. The method according to claim 1 or 2, **characterised by** analysing the hypervariable regions HVR1, HVR2, HVR3 of the mtDNA.

4. The method according to claim 1, **characterised in that** the cell free bodily fluid is urine, plasma or serum.

## Patentansprüche

1. Methode zur nicht invasiven Transplantations- und Transfusionsdiagnose, **gekennzeichnet durch**
a) Entnehmen von zellfreier zirkulierender mitochondrialer DNA (mtDNA) aus einer Probe von zellfreien Körperflüssigkeiten eines Transplantations- oder Transfusionsempfängers;
b) Erkennen, Analysieren und Quantifizieren von mtDNA aus der Probe;
c) Vergleichen der mtDNA aus der Probe mit der mtDNA, welche vom Spender und vom Empfänger vor der Transplantation oder Transfusion erhalten wurde;
d) Verwenden von mtDNA Polymorphien in hypervariablen Regionen, um zwischen der Schädigung im Spendergewebe und der Schädigung im Empfängergewebe in einer Transplantatseinbringung zu unterscheiden, oder das Überleben von zellularen Blutbestandteilen in einer Transfusion zu ermitteln.

2. Methode nach Anspruch 1, **dadurch gekennzeichnet, dass** das Erkennen, Analysieren und Quantifizieren der mtDNA in der Probe des Empfängers durch Taqman Sonden, Molecular Beacons (molekulare Leuchtfeuer) oder andere Mittel von Realzeit PCR, Gene-Array oder SSCP Densitometrie ausgeführt wird.

3. Methode nach Anspruch 1 oder 2, **gekennzeichnet durch** das Analysieren der hypervariablen Regionen HVR1, HVR2, HVR3 der mtDNA.

4. Methode nach Anspruch 1, **dadurch gekennzeichnet, dass** die zellfreien Körperflüssigkeiten Urin, Plasma oder Serum sind.

## Revendications

1. Une méthode pour le diagnostique non-invasif de transplantations et transfusions, **caractérisée en**
a) l'extraction d'ADN mitochondrial (mtADN) non cellulaire circulant d'un échantillon de fluides corporels non cellulaires d'un receveur de transplantation ou de transfusion ;
b) la détection, l'analyse et la quantification du mtADN de l'échantillon;
c) la comparaison du mtADN de l'échantillon avec le mtADN obtenu du donneur et du receveur avant la transplantation ou la transfusion ;
d) l'utilisation de polymorphismes mtADN dans des régions hypervariables afin de distinguer entre le dommage du tissu du donneur et le dommage du tissu du receveur dans une pose de transplant ou afin de déterminer la survie des constituants de sang cellulaires dans une transfusion.

2. La méthode selon la revendication 1, **caractérisée en ce que** la détection, l'analyse et la quantification du mtADN dans l'échantillon du receveur sont faites par des sondes Taqman, des balises moléculaires (molecular beacons) ou d'autres moyens de PCR en temps réel, gene array ou SSCP densitométrie.

3. La méthode selon les revendications 1 ou 2, **caractérisée en** l'analyse des régions hypervariables HVR1, HVR2, HVR3 du mtADN.

4. La méthode selon la revendication 1, **caractérisée en ce que** le fluide corporel non cellulaire est de l'urine, du plasma ou du sérum.
